# EUROPEAN PATENT SPECIFICATION

(11) **EP 4 694 943 B1**
(45) Date of publication and mention of the grant of the patent: **25.11.2026**
(21) Application number: 24724053.4
(22) Date of filing: 11.04.2024
(51) Int. Cl.: A61L 27/34, A61L 27/54, A61L 31/10, A61L 31/16

(54) **MEDICAL DEVICES INCLUDING THERAPEUTIC COATINGS FORMED FROM BIORESORBABLE AND REVERSIBLE THERMO-GELLING EXCIPIENTS**
MEDIZINISCHE VORRICHTUNGEN MIT THERAPEUTISCHEN BESCHICHTUNGEN AUS BIORESORBIERBAREN UND REVERSIBLEN THERMOGELIERENDEN HILFSSTOFFEN
DISPOSITIFS MÉDICAUX COMPRENANT DES REVÊTEMENTS THÉRAPEUTIQUES FORMÉS À PARTIR D'EXCIPIENTS THERMOGÉLIFIANTS BIORÉSORBABLES ET RÉVERSIBLES

(30) Priority: 12.04.2023 US 202363458727 P
(43) Date of publication of application: 18.02.2026
(73) Proprietor: Boston Scientific Scimed Inc., Maple Grove, Minnesota 55311 (US)
(72) Inventor: RO, Andrew J., Plymouth, Minnesota 55446 (US); JOB, Nicholas Michael, Big Lake, Minnesota 55309 (US); FOSS, Aaron Cooper, Plymouth, Minnesota 55447 (US); MAXWELL, Amanda, Minnetonka, Minnesota 55305 (US)
(74) Representative: Vossius & Partner Patentanwälte Rechtsanwälte mbB
(86) International application number: PCT/US2024/024131
(87) International publication number: WO 2024/215928

(56) References cited:
- EP-A1- 3 378 506
- US-A1- 2007 172 509
- US-A1- 2022 193 310

## Description

### TECHNICAL FIELD

The present disclosure pertains to medical devices and more particularly to medical devices that include a therapeutic coating that is formed from bioresorbable and reversible thermo-gelling excipients.

### BACKGROUND

A wide variety of medical devices have been developed for medical use, for example, intravascular use. Some of these devices include guidewires, catheters, balloons, stents, and the like. These devices are manufactured by any one of a variety of different manufacturing methods and may be used according to any one of a variety of methods. Some of these medical devices may include a therapeutic agent. Of the known medical devices and methods, each has certain advantages and disadvantages. There is an ongoing need to provide alternative medical devices as well as alternative methods for manufacturing and using medical devices. This may include the formation of therapeutic agents that have a reduced drug dissolution rate in order to provide a prolonged therapeutic effect.

US 2007/172509 A1 discloses an implantable drug delivery system using a hydrophobic compound as an outer layer or barrier for retarding release of water soluble drugs from the implantable system. The system includes an inner portion of a water soluble drug in a drug matrix material which stabilizes the drug. The outer portion retards the release of the water soluble drug from the inner portion, and includes a hydrophobic non-polymer compound and a binder.

### SUMMARY

The present disclosure pertains to medical devices and more particularly to medical devices that include a therapeutic coating that is formed bioresorbable and reversible thermo-gelling excipients.

In a first example, an elutable coating composition comprises a mixture of excipients comprising a bioresorbable polymer or a thermo-gelling polymer selected from a polycaprolactone (PCL) or poloxamer and a plasticizing agent and a therapeutic agent.

The plasticizing agent comprises acetyl tri-butyl citrate (ATBC) or acetyl trihexyl citrate (ATHC).

Alternatively or additionally to any of the examples above, in another example, the mixture of excipients may comprise polycaprolactone.

Alternatively or additionally to any of the examples above, in another example, the polycaprolactone may have a molecular weight of about 10,000 grams/mole (g/mol) or less.

Alternatively or additionally to any of the examples above, in another example, the mixture of excipients may comprise approximately equal parts polycaprolactone to acetyl tributyl citrate (ATBC).

Alternatively or additionally to any of the examples above, in another example, the mixture of excipients may comprise a poloxamer.

Alternatively or additionally to any of the examples above, in another example, the poloxamer may comprise poloxamer 181 or poloxamer 188.

Alternatively or additionally to any of the examples above, in another example, the mixture of excipients may comprise approximately equal parts poloxamer to acetyl tri-butyl citrate (ATBC).

The therapeutic agent comprises about 60 to about 90 weight percent of the elutable coating composition, the bioresorbable polymer or the thermo-gelling polymer may comprise about 5 to about 20 weight percent of the elutable coating composition, and the plasticizing agent may comprise about 5 to about 20 weight percent of the elutable coating composition.

Alternatively or additionally to any of the examples above, in another example, the therapeutic agent may comprise everolimus.

Alternatively or additionally to any of the examples above, in another example, the elutable coating composition may further comprise an antioxidant.

In another example, a medical device may comprise a medical device component that is at least partially covered by a layer of the elutable coating composition in accordance with any of the examples herein.

Alternatively or additionally to any of the examples above, in another example, the medical device component may be an expandable balloon or an expandable stent.

In another example, an elutable coating composition may comprise an excipient comprising acetyl trihexyl citrate (ATHC) and a therapeutic agent comprising a plurality of everolimus crystals. The ATHC may encapsulate each crystal of the plurality of everolimus crystals.

Alternatively or additionally to any of the examples above, in another example, the therapeutic agent may comprise about 80 to about 95 weight percent of the elutable coating composition and the excipient may comprise about 5 to about 20 weight percent of the elutable coating composition.

Alternatively or additionally to any of the examples above, in another example, the elutable coating composition may further comprise an antioxidant.

In another example, a method of coating a medical device with encapsulated everolimus crystals may comprise forming a coating suspension by suspending everolimus crystals in a first pre-mix that includes acetyl tri-butyl citrate (ATBC), adding a second pre-mix to the first pre-mix, the second pre-mix including a bioresorbable polymer or a thermo-gelling polymer, the bioresorbable polymer or the thermo-gelling polymer mixing with the ATBC and encapsulating the everolimus crystals, thereby forming the coating suspension including the encapsulated everolimus crystals, and applying the coating suspension to the medical device.

Alternatively or additionally to any of the examples above, in another example, once the second pre-mix has been added to the first pre-mix, the bioresorbable polymer or the thermo-gelling polymer and ATBC may be present in approximately equal parts.

Alternatively or additionally to any of the examples above, in another example, the first pre-mix may include cyclohexane.

Alternatively or additionally to any of the examples above, in another example, the second pre-mix may include ethyl acetate.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
FIG. 1 is a schematic side view of an example drug delivery balloon catheter.
FIG. 2 is a cross-sectional view taken through line 2-2 in FIG. 1.
FIG. 3 depicts an example drug-coated stent in a collapsed or delivery configuration.
FIG. 4 depicts an example drug-coated stent in an expanded or deployed configuration.
FIG. 5 schematically depicts a drug coating disposed along an outer or abluminal surface of an example drug-coated stent.
FIG. 6 schematically depicts a drug coating disposed along an inner or luminal surface of an example drug-coated stent.
FIG. 7 schematically depicts the drug coating disposed along both an outer or abluminal surface of an example drug-coated stent and along an inner or luminal surface of the example drug-coated stent.
FIG. 8 is a graphical representation of experimental data.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

The body includes various passageways such as blood vessels and body lumens. These passageways sometimes become occluded (for example, by a tumor or plaque). To widen an occluded body vessel, balloon catheters can be used, for example, in angioplasty. In some embodiments, a balloon catheter can include an inflatable and deflatable balloon carried by a long and narrow catheter body. The balloon can be initially folded around the catheter body to reduce the radial profile of the balloon catheter for easy insertion into the body. During use, the folded balloon can be delivered to a target location in the vessel, for example, a portion occluded by plaque, by threading the balloon catheter over a guide wire previously located in the vessel. The balloon is then inflated, for example, by introducing a fluid (such as a gas or a liquid) into the interior of the balloon. Inflating the balloon can radially expand the vessel so that the vessel can permit an increased rate of blood flow. After use, the balloon is typically deflated and withdrawn from the body. In some instances, it may be desirable to coat, layer, or otherwise apply an elutable drug or therapeutic agent to an outer surface of the balloon to deliver and/or administer the drug or therapeutic agent to a lumen wall when the balloon is expanded. Thus, efficient transfer of the drug coating to the target region and retention of the drug coating at the target region may be desired. It is further contemplated that the drug coating may be required to be durable for ease of handling during the manufacturing process, especially the rewrap process to place a balloon protector over the drug coating. However, the elutable drug coating should balance durability and transferability (e.g., the ability of the drug coating to transfer to the vessel wall), which may be driving by the excipient(s) provided in the coating composition or drug coating. For example, the elutable coating composition or drug coating may be durable enough so that a majority of the drug is retained within the drug coating right before inflation at the target treatment location and can transfer a majority of the drug from the medical device to the target treatment location. Disclosed herein are coating compositions which provide improved durability, transfer efficiency, and drug retention. Further disclosed herein are methods for converting an amorphous form of a material (e.g., a drug) into a crystalline form and methods for encapsulating crystals of the crystalline drug in order to provide an extended release profile for the crystalline drug. In addition, disclosed herein are medical devices with such a coating applied thereto, methods for coating, etc. While the therapeutic coating described herein is discussed relative to balloons and balloon catheters and stents, it is contemplated that the therapeutic coating can be applied to and/or used in conjunction with other medical devices, such as, but not limited to, embolic filters, implantable devices, treatment devices, etc.

The medical device may include an elutable therapeutic coating that is disposed on the surface thereof and including a plurality of everolimus crystals that are encapsulated within a coating that includes a synergistic combination of excipients. A synergistic combination of excipients may be understood to be a combination of excipients that, when used together, provide improved results relative to using either excipient by itself. A synergistic combination of excipients provides better protection for the everolimus crystals, meaning that the everolimus crystals dissolve more slowly over time, providing a delay in drug release or drug dissolution. This provides a tissue drug concentration that lasts longer over time. While the drug coating composition is described as including everolimus, it is contemplated that the drug coating composition may include therapeutic agents in addition to or in place of everolimus.

Surprisingly, it has been found that some combinations of excipients provide better protection than what would be expected by a combination of excipients. For example, in some instances, the synergistic combination of excipients includes polycaprolactone (PCL) and acetyl tri-butyl citrate (ATBC). In another example, the synergistic combination of excipients includes poloxamer and acetyl tri-butyl citrate (ATBC). In some cases, the excipient may include acetyl trihexyl citrate (ATHC). In yet another example, the synergistic combination of excipients includes polycaprolactone and acetyl tri-butyl citrate (ATHC). In a further example, the synergistic combination of excipients includes poloxamer and acetyl tri-butyl citrate (ATHC). In yet another example, the synergistic combination of excipients includes ethyl cellulose and acetyl tri-butyl citrate (ATHC).

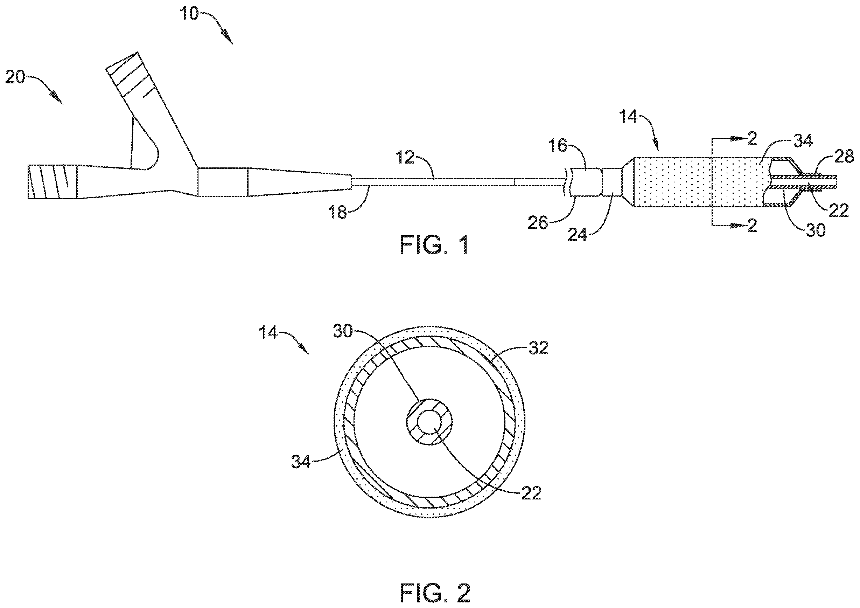

FIG. 1 is a schematic side view of a drug delivery balloon catheter 10. A cross-sectional view of the drug delivery balloon catheter 10 is shown in FIG. 2. In the illustrated embodiment, the catheter 10 may include an elongated shaft 12, an inflatable balloon 14 coupled at or to a distal portion 16 of the shaft 12, along with other components. The elongated shaft 12 may include a tubular member having a proximal portion 18, and one or more lumens extending between the proximal portion 18 and the distal portion 16. The elongated shaft 12 may be configured to have a substantially circular cross-section; however, it may be configured to have other suitable cross-sectional shapes, such as elliptical, oval, polygonal, irregular, etc. In addition, the elongated shaft 12 may be flexible along its entire length or adapted for flexure only along portions of its length. The required degree of flexibility of the elongated shaft 12 may be predetermined based on its intended navigation to a target vascular passage, and the amount of inertial force required for advancing the elongated shaft 12 through the vascular passage. The catheter 10 may be configured as an over-the-wire (OTW) catheter, a single-operator exchange (SOE) catheter, a fixed wire catheter, and/or the like.

The cross-sectional dimensions of the elongated shaft 12 may vary according to the desired application. Generally, the cross-sectional dimensions of the elongated shaft 12 may be sized smaller than the typical blood vessel in which the catheter 10 is to be used. The length of the elongated shaft 12 may vary according to the location of the vascular passage where drug delivery is desired. In some instances, a 6F or a 5F catheter may be used as the elongated shaft 12, where "F," also known as French catheter scale, is a unit to measure catheter diameter (1F = 1/3 millimeter (mm)). In addition, the elongated shaft 12 or a portion thereof may be selectively steerable. Mechanisms such as, pull wires and/or other actuators may be used to selectively steer the elongated shaft 12, if desired.

The proximal portion 18 of the elongated shaft 12 may include a handle 20 usable to manually maneuver the distal portion 16 of the elongated shaft 12. The handle 20 may include one or more ports that may be used to introduce any suitable medical device, fluid or other interventions. For example, the handle 20 may include a guidewire port in communication with a guidewire lumen 22 (shown in the cut-away portion at the distal end of the catheter 10 and also in FIG. 2) which may be used to introduce a guidewire having an appropriate thickness into the elongated shaft 12, which may guide the elongated shaft 12 to the target location within an artery. Furthermore, the handle 20 may include an inflation port configured to be coupled to a source of inflation fluid for delivering an inflation fluid through an inflation lumen of the catheter shaft 12 to the inflatable balloon 14. In certain embodiments, the elongated shaft 12 may include one or more additional lumens, which may be configured for a variety of purposes, such as delivering medical devices or for providing fluids, such as saline, to a target location.

The inflatable balloon 14 may be operably coupled at or to the distal portion 16 of the elongated shaft 12. In particular, a proximal portion or waist 24 of the inflatable balloon 14 may be secured to the distal portion 16 of the elongated shaft 12, such as an outer tubular member 26 of the elongated shaft 12. Furthermore, a distal portion or waist 28 of the inflatable balloon 14 may be secured to the distal portion 16 of the elongated shaft 12, such as an inner tubular member 30 of the elongate shaft 12 extending through the outer tubular member 26. A suitable securing method(s) may be employed to couple the two structures, including but not limited to adhesive bonding, thermal bonding (e.g., hot jaws, laser welding, etc.) or other bonding technique, as desired. The inflatable balloon 14 may be configured to be expanded from a deflated state to an inflated state through delivery of an inflation fluid (e.g., saline) through the inflation lumen of the catheter shaft 12. The balloon 14 may be deflated during introduction of the catheter inside the patient's body, whereas the balloon 14 may be inflated once it reaches the target site within the body vessel.

The inflatable balloon may be manufactured using or otherwise formed of any suitable material, including polymer materials, such as polyamide, polyether block amide (PEBA), polyester, nylon, etc. The inflatable balloon 14 may have a substantially cylindrical configuration with a circular cross-section, as shown in the illustrative embodiment. However, in other embodiments the inflatable balloon 14 may have another suitable configuration or shape, if desired.

The inflatable balloon 14 may include a balloon wall 32 have a drug coating 34 disposed thereon. In some cases, the drug coating or coating composition 34 may include encapsulated crystalline everolimus, as will be described in more detail herein. The drug coating 34 may be disposed along substantially the entire length and/or circumference of the balloon 14 or along one or more portions of the balloon 14. For example, the drug coating 34 may be disposed along a central or body portion of the balloon 14. The drug coating 34 disposed on the balloon 14/balloon wall 32 may have an average thickness in the range of about 1 micron to about 50 microns, for example.

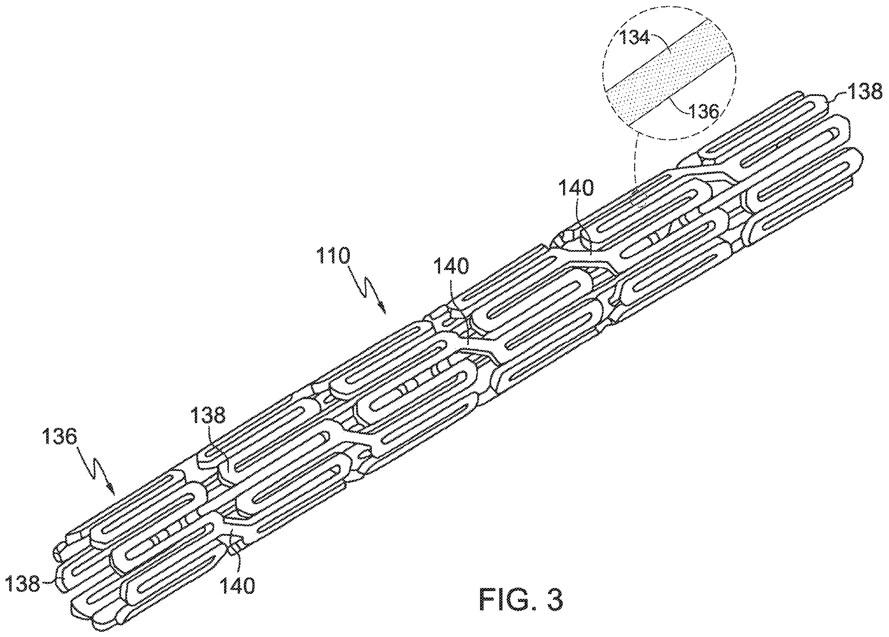

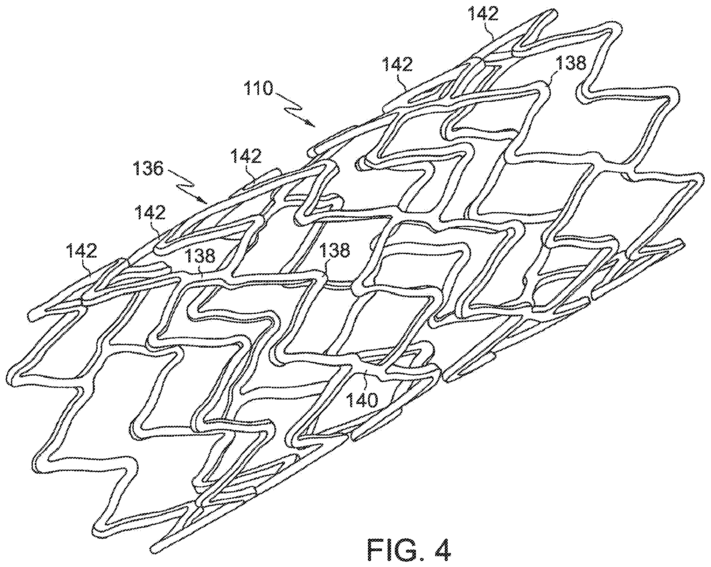

FIGS. 3-4 illustrate an example drug-coated stent 110 in either a collapsed or delivery configuration (FIG. 3) or in an expanded or deployed configuration (FIG. 4). In general, the stent 110 may be delivered to a suitable target region via a catheter/delivery system while in the collapsed configuration. Upon reaching the target region, the stent 110 may expand or be expanded into the expanded configuration. The stent 110 may be self-expanding (e.g., the stent 110 may be formed from a shape memory material such as nitinol) or may be balloon expandable. When the stent 110 is self-expanding, the stent 110 may be held/constrained in the collapsed configuration during delivery and then unconstrained to allow the stent 110 to expand (e.g., self-expand) to the expanded configuration. When the stent 110 is balloon-expandable, the stent 110 may be constrained crimped onto a delivery device/catheter and then expanded (e.g., via an expandable member or balloon) when at/adjacent the target region.

The stent 110 may include strut framework 136. The strut framework 136 may define one or more peaks or apex regions 138 and/or one or more connecting regions 140. Intermediate regions 142 may interconnect the apex regions 138 and/or the connecting regions 140. In some instances, the stent 110 may be formed from a cut (e.g., laser cut) tube. In such instances, the structural arrangement of the apex regions, connecting regions 140, and/or intermediate regions 142 may be determined by the cut pattern. It can be appreciated that a wide variety of patterns/arrangement may be used for the stent 110. In other instances, the stent 110 may be a woven or braided stent, a stent formed from a flat sheet of material that is rolled into a stent formation, molded, cast, and/or the like.

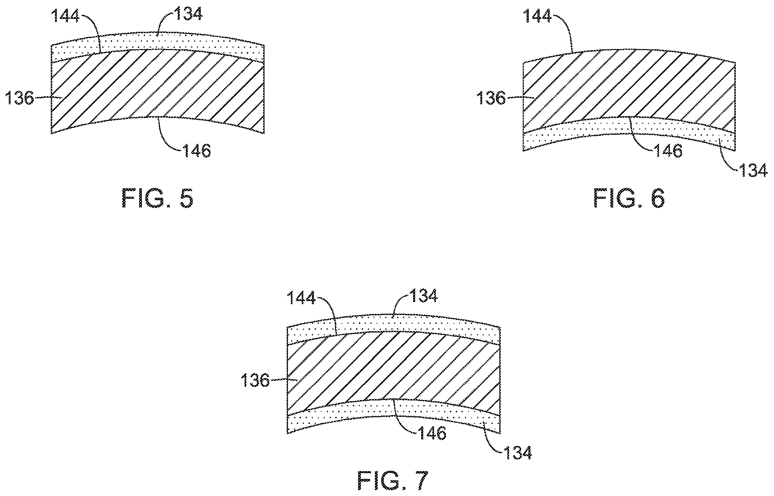

A drug coating or coating composition 134 may be disposed along the strut framework 136. For example, FIG. 5 schematically depicts the drug coating 134 disposed along an outer or abluminal surface 144 of the stent 110 (e.g., along the outer or abluminal surface 144 of the strut framework 136). FIG. 6 schematically depicts the drug coating 134 disposed along an inner or luminal surface 146 of the stent 110 (e.g., along the inner or luminal surface 146 of the strut framework 136). FIG. 7 schematically depicts the drug coating 134 along both an outer or abluminal surface 144 of the stent 110 and the inner or luminal surface 146 of the stent 110 (e.g., along both the outer or abluminal surface 144 of the strut framework 136 and the inner or luminal surface 146 of the strut framework 136). While not explicitly shown, in some examples, the drug coating 134 may be additionally or alternatively disposed along the lateral sides of the strut framework 136, the lateral sides extending between the outer or abluminal surface 144 and the inner or luminal surface 146. It is noted that FIGS. 5-7 are intended to represent, schematically, a cross-sectional view of a portion (e.g., a singular region or portion of a strut) of the strut framework 136. In these views, the strut framework 136 is depicted as being partially arcuate in shape. This is intended to correspond to the generally cylindrical shape of the stent 110, for example when the stent 110 is in the expanded configuration. In some cases, the drug coating 134 may include encapsulated crystalline everolimus as will be described in more detail herein. The drug coating 134 disposed on the strut framework 136 may have an average thickness in the range of about 1 micron to about 50 microns, for example.

The terms "therapeutic agents," "drugs," "bioactive agents," "pharmaceuticals," "pharmaceutically active agents", and other related terms may be used interchangeably herein and include genetic therapeutic agents, non-genetic therapeutic agents, and cells. Therapeutic agents may be used singly or in combination. A wide range of therapeutic agent loadings can be used in conjunction with the devices of the present invention, with the pharmaceutically effective amount being readily determined by those of ordinary skill in the art and ultimately depending, for example, upon the condition to be treated, the nature of the therapeutic agent itself, the tissue into which the dosage form is introduced, and so forth.

The drug coating 34, 134 may include a therapeutic agent that includes everolimus. However, other beneficial agents may include anti-thrombotic agents, anti-proliferative agents, anti-inflammatory agents, anti-migratory agents, agents affecting extracellular matrix production and organization, antineoplastic agents, anti-mitotic agents, anesthetic agents, anti-coagulants, vascular cell growth promoters, vascular cell growth inhibitors, cholesterol-lowering agents, vasodilating agents, and agents that interfere with endogenous vasoactive mechanisms.

More specific drugs or therapeutic agents include paclitaxel, rapamycin, sirolimus, everolimus, tacrolimus, heparin, diclofenac, aspirin, Epo D, dexamethasone, estradiol, halofuginone, cilostazol, geldanamycin, ABT-578 (Abbott Laboratories), trapidil, liprostin, actinomycin D, Resten-NG, Ap-17, abciximab, clopidogrel, Ridogrel, beta-blockers, bARKct inhibitors, phospholamban inhibitors, and SERCA 2 gene/protein, resiquimod, imiquimod (as well as other imidazoquinoline immune response modifiers), human apolipoproteins (e.g., AI, AII, AIII, AIV, AV, etc.), vascular endothelial growth factors (e.g., VEGF-2), as well as derivatives of the forgoing, among many others.

In some embodiments, the drug may be a macrolide immunosuppressive (limus) drug. In some embodiments, the macrolide immunosuppressive drug is rapamycin, biolimus (biolimus A9), 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin, 40-O-(2-Hydroxy)ethoxycarbonylmethyl-rapamycin, 40-O-(3-Hydroxy)propyl-rapamycin, 40-O-(6-Hydroxy)hexyl-rapamycin, 40-O-[2-(2-Hydroxy)ethoxy]ethyl-rapamycin, 40-O-[(3S)-2,2-Dimethyldioxolan-3-yl]methyl-rapamycin, 40-O-[(2S)-2,3-Dihydroxyprop-1-yl]-rapamycin, 40-O-(2-Acetoxy)ethyl-rapamycin, 40-O-(2-Nicotinoyloxy)ethyl-rapamycin, 40-O-[2-(N-Morpholino)acetoxy]ethyl-rapamycin, 40-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 40-O-(2-Aminoethyl)-rapamycin, 40-O-(2-Acetaminoethyl)-rapamycin, 40-O-(2-Nicotinamidoethyl)-rapamycin, 40-O-(2-(N-Methyl-imidazo-2'-ylcarbethoxamido)ethyl)-rapamycin, 40-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), or derivative, isomer, racemate, diastereoisomer, prodrug, hydrate, ester, or analog thereof. Other drugs may include anti-inflammatory agents such as dexamethasone, prednisolone, corticosterone, budesonide, estrogen, sulfasalazine, mesalamine, and analogues thereof; antineoplastic/antiproliferative/anti-miotic agents such as paclitaxel, 5-fluorouracil, cisplatin, vinblastine, vincristine, epothilones, endostatin, angiostatin, thymidine kinase inhibitors, and analogues thereof; anesthetic agents such as lidocaine, bupivacaine, ropivacaine, and analogues thereof; anti-coagulants; and growth factors.

In some cases, everolimus may be the drug used. Everolimus, which is also known as 40-O-(2-Hydroxyethyl)rapamycin, has the following chemical structure:

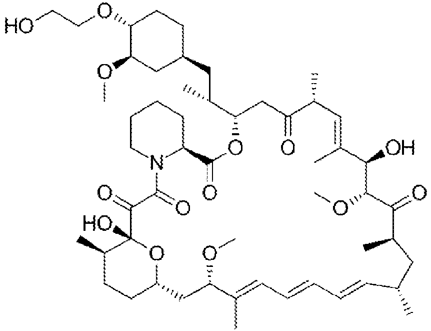

In some instances, providing a drug coated medical device 10, 110 with a drug coating 34, 134 that is adapted to permit an extended-release profile may be beneficial in treating small vessel occlusions and/or vascular disease. In some instances, improved results may be achieved in cases where the extended-release profile means that a useful fraction of the drug remains for an extended period of time, thereby increasing the efficacy of the drug in treating whatever condition is being treated, at least in part because a useful fraction of the drug remains for a longer period of time. In some instances, a drug coating with an extended-release profile may mean that not as much drug is required in the coating in order to achieve a desired effect, for example.

In some instances, a therapeutic coating including encapsulated everolimus crystals may provide a 30-day tissue everolimus concentration of at least 0.5 nanograms of everolimus per milligram of tissue. In some cases, a therapeutic coating including encapsulated everolimus crystals may provide a 30-day tissue everolimus concentration of at least 0.6 nanograms of everolimus per milligram of tissue. In some cases, a therapeutic coating including encapsulated everolimus crystals may provide a 30-day tissue everolimus concentration of at least 0.7 nanograms of everolimus per milligram of tissue. In some cases, a therapeutic coating including encapsulated everolimus crystals may provide a 30-day tissue everolimus concentration of at least 0.8 nanograms of everolimus per milligram of tissue. In some cases, a therapeutic coating including encapsulated everolimus crystals may provide a 30-day tissue everolimus concentration of at least 0.9 nanograms of everolimus per milligram of tissue. In some cases, a therapeutic coating including encapsulated everolimus crystals may provide a 30-day tissue everolimus concentration of at least 1 nanogram of everolimus per milligram of tissue.

In some instances, the drug coating may include individual drug particles that are encapsulated with one or more excipients. The drug particles may include crystals of the drug, for example. Drug crystals may be formed in a variety of ways, for example. In some cases, a drug or other therapeutic agent may be available in an amorphous form, and a variety of processes may be used to convert an amorphous drug or other therapeutic agent into a crystalline drug or other therapeutic agent. One illustrative process for converting an amorphous drug such as amorphous everolimus into a crystalline drug such as crystalline everolimus is described herein.

A medical device 10, 110, or portions thereof, may be coated with a therapeutic composition 34, 134. As an example, the therapeutic composition may include everolimus. Everolimus crystals may be coated with a mixture of excipients in order to form encapsulated everolimus crystals that are suspended in a coating composition 34, 134. In some instances, the medical device 10, 110, or portions thereof, may be contacted with the coating composition in order to form a coating on the medical device. In some instances, the medical device 10, 110, or a portion thereof, may be dipped into the coating composition. In some cases, vapor deposition may be used to transfer the coating composition to the medical device 10, 110. In some cases, a roller coating process may be used to transfer the coating composition to the medical device 10, 110. These are just examples. In some cases, the coating composition may be sprayed onto the medical device 10, 110, or may be sprayed onto a particular portion or region of the medical device 10, 110.

When the medical device 10 includes an inflatable balloon 14, for example, the coating composition may be sprayed onto at least a portion of outer surface of the inflatable balloon 14 in order to be able to subsequently transfer at least a portion of the drug coating 34 to blood vessel walls. Alternative coating processes may be used such as dip coating, roller coating, vapor deposition, and/or the like, and/or other suitable coating processes. There may be little or no benefit to applying the coating composition to other portions of the medical device such as a balloon catheter shaft because the balloon catheter shaft may make incidental contact at best with the blood vessel walls, for example.

When the medical device is or includes an expandable stent 110, the coating composition may be sprayed onto at least a portion of the outer surface 144 of the expandable stent 110 in order to be able to subsequently transfer at least a portion of the drug coating to blood vessel walls. There may be reduced benefit to applying the coating composition to an interior surface 146 of the expandable stent 110 because the interior surface of the expandable stent 110 is unlikely to contact the blood vessel walls. There may be some benefit to transferring at least a portion of the drug coating to blood flowing through the expandable stent, as the blood will subsequently contact the blood vessel walls, particularly downstream of where the expandable stent 110 is deployed. Thus, the coating composition may be applied to the interior surface 146 and/or the lateral sidewalls of the expandable stent 110.

In additional to the therapeutic agent, the coating composition 34, 134 may further include one or more excipients. Excipients can be used to enhance the durability of the drug coating 34, 134, facilitate drug transfer to the lesion, and/or control drug dissolution. An example excipient may include polycaprolactone (PCL) which is a biodegradable polyester. PCL has the following chemical structure:

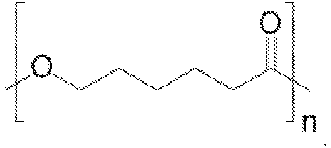

Another example excipient may include acetyl tri-butyl citrate (ATBC), which in some cases may be referred to by its IUPAC name of tributyl 2-acetyloxypropane-1,2,3-tricarboxylate. ATBC has the following chemical structure:

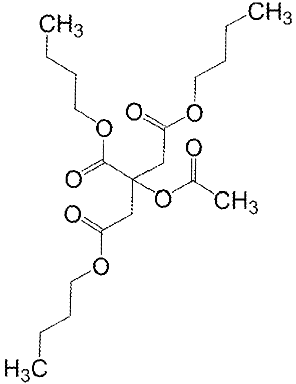

Another example excipient may include poloxamers, which are nonionic triblock copolymers. The triblock may include a central chain of hydrophobic polyoxypropylene (poly(propylene oxide)) with chains of hydrophilic polyoxyethylene (poly(ethylene oxide)) positioned on either side thereof. The lengths of the polymer blocks can be varied or customized to result in a number of different poloxamers having varying properties. Poloxamer may be more commonly called Pluronic^{®} (manufactured by BASF, Ludwigshafen, Germany). Poloxamer may have the following generic chemical structure: 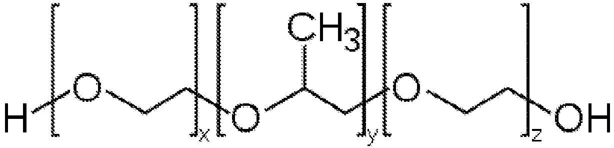 where x, y, and z are number of repeating units (e.g., degree of polymerization) of each block.

Another example excipient may include acetyl trihexyl citrate (ATHC), which in some cases may be referred to by its IUPAC name of trihexyl 2-(acetyloxy)propane-1,2,3-tricarboxylate. ATHC has the following chemical structure:

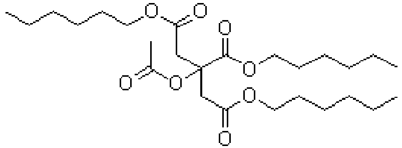

In some cases, one or excipients may optionally include two or more excipients or a mixture of excipients. In a first example, the mixture of excipients may include PCL and ATBC. In some examples, additional components may also be used in the coating composition 34, 134. In some cases, an antioxidant, such as, but not limited to, butylated hydroxytoluene (BHT) may be used in the composition. As an example, coating everolimus crystals with a mixture of excipients to form encapsulated everolimus crystals suspended in a coating composition may include suspending everolimus crystals in a first pre-mix that includes ATBC. A second pre-mix may be added to the first pre-mix, the second pre-mix including PCL. When the second pre-mix including PCL is added to the first pre-mix that includes the suspended everolimus crystals and ATBC, the PCL mixes with the ATBC and coats the everolimus crystals. This forms a coating composition that includes encapsulated everolimus crystals held within a suspension. In some examples, individual everolimus crystals may have a coating (e.g., the coating formed from PCL and ATBC) that is less than 1 micron thick.

The PCL may have a molecular weight in the range of about 10,000 grams/mole (g/mol) or less. It is contemplated that PCL having a molecular weight of less than 10,000 g/mol may increase the tendency of the PCL to stay in solution and/or dispersible in an organic solvent system that would also disperse everolimus.

In some instances, the encapsulated everolimus crystals may be considered as including from 0 to 30 weight percent ATBC, from 0 to 30 weight percent PCL and from 70 to 100 weight percent everolimus. If so provided, BHT has a weight percent of 0.5% or less of the weight of the everolimus (or therapeutic agent). In some instances, the encapsulated everolimus crystals may be considered as including from 5 to 20 weight percent ATBC, from 5 to 20 weight percent PCL and from 60 to 90 weight percent everolimus. In some instances, the encapsulated everolimus crystals may include about 80 weight percent everolimus and about 20 weight percent excipient, with the excipient being a combination of ATBC and PCL. As an example, the excipient may be about equal parts of ATBC and PCL. In other examples, the excipient may have a weight ratio of in the range of about 1:10 ATBC to PCL to about 10:1 ATBC to PCL. In an illustrative example, the final coating composition may have a weight ratio of 8:1:1 of therapeutic agent to ATBC to PCL.

In some instances, the first pre-mix may include a singular solvent, and preparing the first pre-mix may include placing the solvent in a suitable container and adding the crystalline everolimus and the ATBC. When the solvent includes a mixture of materials, preparing the first pre-mix may include combining or mixing two or more solvents prior to adding the crystalline everolimus and the ATBC. In some examples, the ATBC may be mixed with the solvent prior to adding the ATBC/solvent pre-mix to the therapeutic agent. This may help prevent the therapeutic agent from fully dissolving in the first pre-mix.

In at least some instances, the solvent used for forming the first pre-mix may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic or alicyclic hydrocarbons such as, but not limited to, hexane, heptane, cyclohexane, or the like; aromatic hydrocarbons, such as, but not limited to, toluene or xylenes; and ester solvents such as, but not limited to, ethyl acetate. Mixed solvents such as, but not limited to, ethyl acetate/heptane, acetone/water, IPA/water, IPA/THF, methanol/water, IPA/heptane, or THF/heptane can also be used, for example. Other solvent systems are also contemplated. In some cases, the solvent used for forming the first pre-mix may include cyclohexane.

In some embodiments, the process of forming the first pre-mix may take place at ambient temperature and at atmospheric pressure. In some instances, the first pre-mix may be formed as part of the process of converting the amorphous everolimus into crystalline everolimus. Alternatively, or additionally, ATBC may be added to the suspension that results from the crystallization process, for example. In yet other examples, the first pre-mix may be formed by first mixing the ATBC with the solvent(s) and subsequently adding the crystalline everolimus (or adding the ATBC/solvent pre-mix to the crystalline everolimus).

In some instances, the first pre-mix may include crystalline everolimus suspended in a solvent such as cyclohexane. Alternatively, other solvents such as isopropanol, ethanol or similar alcohols, acetone, tetrahydrofuran, or acetonitrile may be used. In some instances, the first pre-mix may also include other additives such as, but not limited to, BHT, for example. BHT may prevent degradation of the therapeutic agent prior to deploying the coating 34, 134 in the body. It will be appreciated that the crystalline everolimus may minimally dissolve in the solvent, and thus will be a suspension. As such, the crystalline everolimus may maintain its crystal size when suspended in the first solvent. The ATBC should at least partially dissolve in the solvent.

In some instances, the second pre-mix may include a singular solvent, and preparing the second pre-mix may include placing the solvent in a suitable container and adding the PCL. When the solvent includes a mixture of materials, preparing the second pre-mix may include combining or mixing two or more solvents prior to adding the PCL.

In at least some instances, the solvent used for forming the second pre-mix may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic hydrocarbons such as, but not limited to, hexane, heptane, or the like; aromatic hydrocarbons, such as, but not limited to, toluene or xylenes; and ester solvents such as, but not limited to, ethyl acetate. Mixed solvents such as ethyl acetate/heptane, acetone/water, IPA/water, methanol/water, IPA/heptane, or THF/heptane can also be used, for example. Other solvent systems are also contemplated. In some cases, the solvent used for forming the second pre-mix may include ethyl acetate.

In some instances, the process of forming the second pre-mix may take place at ambient temperature and at atmospheric pressure. The second pre-mix may include PCL and one or more solvents. In some instances, the second pre-mix may include PCL dissolved in a solvent such as ethyl acetate, although other solvents such as heptane or similar alkanes, or even water, may be used. The PCL will dissolve in the ethyl acetate, providing a solution.

It is contemplated that at least one of the first or second pre-mixes may include a polar solvent and the other of the first or second pre-mixes may include a non-polar solvent. For example, the first pre-mix may include cyclohexane which is a non-polar solvent and the second pre-mix may include ethyl acetate which is a polar solvent. The combination of a polar and a non-polar solvent may help to disperse the everolimus crystals. A dispersion of the everolimus crystals may help to dispense controlled, consistent amounts on the medical device to yield a drug coating composition. The organic solvents (e.g., cyclohexane and ethyl acetate in the example given above) are evaporated during the coating manufacturing process. Thus, the drug coating 34, 134 on the medical device may include a drug, one or more excipients, and, optionally, an antioxidant.

In some cases, after the first pre-mix and the second pre-mix have been formed, the second pre-mix may be added to the first pre-mix. In another example, the crystalline everolimus may be added to a container, then the first pre-mix including ATBC, and any other additives may be added to the container including the everolimus. Next, the second pre-mix including the PCL may be added to the everolimus/ATBC mixture. As a result, the ATBC and the PCL mix together and coat the individual crystals of everolimus, resulting in encapsulated everolimus crystals. In some cases, the process may occur quickly. In some cases, the process may occur more slowly. The first pre-mix may include a volume of cyclohexane and the second pre-mix may include a volume of ethyl acetate to provide a ratio of ethyl acetate to cyclohexane in the range of about 1 to 4 to about 1 to 10 when the second pre-mix is added to the first pre-mix. It will be appreciated that having too much everolimus solubility will dissolve the everolimus crystals. It will be appreciated that the stated process of forming the first pre-mix, forming the second pre-mix and then combining the first pre-mix and the second pre-mix is merely illustrative, and other processes are contemplated.

Surprisingly, it has been found that some combinations of excipients provide better protection than what would be expected by a combination of excipients. For example, in some instances, the synergistic combination of excipients includes polycaprolactone (PCL). In these and other instances, the synergistic combination of excipients include acetyl tri-butyl citrate (ATBC). In an example, the synergistic combination of excipients may include one-part polycaprolactone (PCL) to one-part acetyl tri-butyl citrate (ATBC). The PCL is a bioresorbable excipient that may provide coating durability, adhesion to vessel wall allowing for a higher residence time of the drug coating 34, 134, and a dissolution barrier (e.g., inhibits or slows the dissolution of the therapeutic agent). The PCL may resorb into the body, over time. The ATBC may be a plasticizer or plasticizing agent which hardens the coating composition and may slow the dissolution of the therapeutic agent.

In another example, the mixture of excipients may include poloxamer and ATBC. In some examples, additional components may also be used in the coating composition 34, 134. In some cases, an antioxidant, such as, but not limited to, butylated hydroxytoluene (BHT) may be used in the composition. As an example, coating everolimus crystals with a mixture of excipients to form encapsulated everolimus crystals suspended in a coating composition may include suspending everolimus crystals in a first pre-mix that includes ATBC. A second pre-mix may be added to the first pre-mix, the second pre-mix including poloxamer. When the second pre-mix including poloxamer is added to the first pre-mix that includes the suspended everolimus crystals and ATBC, the poloxamer mixes with the ATBC and coats the everolimus crystals. This forms a coating composition that includes encapsulated everolimus crystals held within a suspension. In some examples, individual everolimus crystals may have a coating (e.g., the coating formed from poloxamer and ATBC) that is less than 1 micron thick.

In some cases, the poloxamer may be poloxamer 181 or poloxamer 188. Typically, the first two digits of the number that follows poloxamer multiplied by 100 gives the molecular mass of the polyoxypropylene core and the last digit multiplied by 10 gives the percentage of polyoxyethylene content. Thus, poloxamer 181 would include polyoxypropylene having a molecular mass of 1800 (e.g., 18*100) and would also include 10% polyoxyethylene (e.g., 1*10) and poloxamer 188 would include polyoxypropylene having a molecular mass of 1800 (e.g., 18*100) and would also include 80% polyoxyethylene (e.g., 8*10) However, in some cases other grades of poloxamer may be used, as desired. It is contemplated that poloxamers having a lower molecular weight may increase the tendency of the poloxamer to stay in solution and/or dispersible in an organic solvent system that would also disperse everolimus. For example, the poloxamer may have a molecular weight of less than 10,000 daltons (Da). Poloxamers are known to gel at body temperatures. It is contemplated that the gelled poloxamer may act as a diffusion barrier. It is further contemplated that the gelling may be thermoreversible.

In some instances, the encapsulated everolimus crystals may be considered as including from 0 to 30 weight percent ATBC, from 0 to 30 weight percent poloxamer and from 70 to 100 weight percent everolimus. If so provided, BHT has a weight percent of 0.5% or less of the weight of the everolimus (or therapeutic agent). In some instances, the encapsulated everolimus crystals may be considered as including from 5 to 20 weight percent ATBC, from 5 to 20 weight percent poloxamer and from 60 to 90 weight percent everolimus. Compositions within these ranges were found to have improved dissolution rates relative to formulations that only used ATBC as a single excipient. In some instances, the encapsulated everolimus crystals may include about 80 weight percent everolimus and about 20 weight percent excipient, with the excipient being a combination of ATBC and poloxamer. As an example, the excipient may be about equal parts of ATBC and poloxamer. In other examples, the excipient may have a weight ratio of in the range of about 1:10 ATBC to poloxamer to about 10:1 ATBC to poloxamer. In an illustrative example, the final coating composition may have a weight ratio of 8:1:1 of therapeutic agent to ATBC to poloxamer.

In some instances, the first pre-mix may include a singular solvent, and preparing the first pre-mix may include placing the solvent in a suitable container and adding the crystalline everolimus and the ATBC. When the solvent includes a mixture of materials, preparing the first pre-mix may include combining or mixing two or more solvents prior to adding the crystalline everolimus and the ATBC. In some examples, the ATBC may be mixed with the solvent prior to adding the ATBC/solvent pre-mix to the therapeutic agent. This may help prevent the therapeutic agent from fully dissolving in the first pre-mix.

In at least some instances, the solvent used for forming the first pre-mix may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic or alicyclic hydrocarbons such as, but not limited to, hexane, heptane, cyclohexane, or the like; aromatic hydrocarbons, such as, but not limited to, toluene or xylenes; and ester solvents such as, but not limited to, ethyl acetate. Mixed solvents such as, but not limited to, ethyl acetate/heptane, acetone/water, IPA/water, IPA/THF, methanol/water, IPA/heptane, or THF/heptane can also be used, for example. Other solvent systems are also contemplated. In some cases, the solvent used for forming the first pre-mix may include cyclohexane.

In some embodiments, the process of forming the first pre-mix may take place at ambient temperature and at atmospheric pressure. In some instances, the first pre-mix may be formed as part of the process of converting the amorphous everolimus into crystalline everolimus. Alternatively, or additionally, ATBC may be added to the suspension that results from the crystallization process, for example. In yet other examples, the first pre-mix may be formed by first mixing the ATBC with the solvent(s) and subsequently adding the crystalline everolimus (or adding the ATBC/solvent pre-mix to the crystalline everolimus).

In some instances, the first pre-mix may include crystalline everolimus suspended in a solvent such as cyclohexane. Alternatively, other solvents such as isopropanol, ethanol or similar alcohols, acetone, tetrahydrofuran, or acetonitrile may be used. In some instances, the first pre-mix may also include other additives such as, but not limited to, BHT, for example. BHT may prevent degradation of the therapeutic agent prior to deploying the coating 34, 134 in the body. It will be appreciated that the crystalline everolimus may minimally dissolve in the solvent, and thus will be a suspension. As such, the crystalline everolimus may maintain its crystal size when suspended in the first solvent. The ATBC should at least partially dissolve in the solvent.

In some instances, the second pre-mix may include a singular solvent, and preparing the second pre-mix may include placing the solvent in a suitable container and adding the poloxamer. When the solvent includes a mixture of materials, preparing the second pre-mix may include combining or mixing two or more solvents prior to adding the poloxamer.

In at least some instances, the solvent used for forming the second pre-mix may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic hydrocarbons such as, but not limited to, hexane, heptane, or the like; aromatic hydrocarbons, such as, but not limited to, toluene or xylenes; and ester solvents such as, but not limited to, ethyl acetate. Mixed solvents such as ethyl acetate/heptane, acetone/water, IPA/water, methanol/water, IPA/heptane, or THF/heptane can also be used, for example. Other solvent systems are also contemplated. In some cases, the solvent used for forming the second pre-mix may include ethyl acetate.

In some instances, the process of forming the second pre-mix may take place at ambient temperature and at atmospheric pressure. The second pre-mix may include poloxamer and one or more solvents. In some instances, the second pre-mix may include poloxamer dissolved in a solvent such as ethyl acetate, although other solvents such as heptane or similar alkanes, or even water, may be used. The poloxamer will dissolve in the ethyl acetate, providing a solution.

It is contemplated that at least one of the first or second pre-mixes may include a polar solvent and the other of the first or second pre-mixes may include a non-polar solvent. For example, the first pre-mix may include cyclohexane which is a non-polar solvent, and the second pre-mix may include ethyl acetate which is a polar solvent. The combination of a polar and a non-polar solvent may help to disperse the everolimus crystals. A dispersion of the everolimus crystals may help to dispense controlled, consistent amounts on the medical device to yield a drug coating composition. The organic solvents (e.g., cyclohexane and ethyl acetate in the example given above) are evaporated during the coating manufacturing process. Thus, the drug coating 34, 134 on the medical device may include a drug, one or more excipients, and, optionally, an antioxidant.

In some cases, after the first pre-mix and the second pre-mix have been formed, the second pre-mix may be added to the first pre-mix. In another example, the crystalline everolimus may be added to a container, then the first pre-mix including ATBC, and any other additives, may be added to the container including the everolimus. Next, the second pre-mix including the poloxamer may be added to the everolimus/ATBC mixture. As a result, the ATBC and the poloxamer mix together and coat the individual crystals of everolimus, resulting in encapsulated everolimus crystals. In some cases, the process may occur quickly. In some cases, the process may occur more slowly. The first pre-mix may include a volume of cyclohexane and the second pre-mix may include a volume of ethyl acetate to provide a ratio of ethyl acetate to cyclohexane in the range of about 1 to 4 to about 1 to 10when the second pre-mix is added to the first pre-mix. It will be appreciated that having too much everolimus solubility will dissolve the everolimus crystals. It will be appreciated that the stated process of forming the first pre-mix, forming the second pre-mix and then combining the first pre-mix and the second pre-mix is merely illustrative, and other processes are contemplated.

Surprisingly, it has been found that some combinations of excipients provide better protection than what would be expected by a combination of excipients. For example, in some instances, the synergistic combination of excipients includes poloxamer. In these and other instances, the synergistic combination of excipients include acetyl tri-butyl citrate (ATBC). In an example, the synergistic combination of excipients may include one-part poloxamer to one-part acetyl tri-butyl citrate (ATBC). The poloxamer is a reversible thermo-gelling excipient that may provide coating durability, adhesion to vessel wall allowing for a higher residence time of the drug coating 34, 134, and a dissolution barrier (e.g., inhibits or slows the dissolution of the therapeutic agent). The poloxamer may resorb into the body over time. The ATBC may be a plasticizer or plasticizing agent which hardens the coating composition and may slow the dissolution of the therapeutic agent.

In another example, the excipient may include acetyl trihexyl citrate (ATHC). ATHC may be a plasticizer or plasticizing agent which hardens the coating composition and may slow the dissolution of the therapeutic agent. As ATHC is more non-polar compared to ATBC, the ATHC may slow the dissolution of the therapeutic agent to a greater degree than ATBC. In some examples, additional components may also be used in the coating composition 34, 134. In some cases, an antioxidant, such as, but not limited to, butylated hydroxytoluene (BHT) may be used in the composition. As an example, coating everolimus crystals with an excipient to form encapsulated everolimus crystals suspended in a coating composition may include suspending everolimus crystals in a first pre-mix that includes ATHC. A second pre-mix or second solvent may be added to the first pre-mix. When the second pre-mix is added to the first pre-mix that includes the suspended everolimus crystals and ATHC, the everolimus crystals are coated with ATHC. This forms a coating composition that includes encapsulated everolimus crystals held within a suspension. In some examples, individual everolimus crystals may have a coating (e.g., the coating formed from ATHC) that is less than 1 micron thick.

In some instances, the encapsulated everolimus crystals may be considered as including from about 0 to about 30 weight percent ATHC and from about 70 to about 100 weight percent everolimus. If so provided, BHT has a weight percent of 0.5% or less of the weight of the everolimus (or therapeutic agent). In some instances, the encapsulated everolimus crystals may be considered as including from about 5 to 20 weight percent ATHC and from about 80 to 95 weight percent everolimus. Compositions within these ranges were found to have improved dissolution rates relative to formulations that only used ATBC as a single excipient. In some instances, the encapsulated everolimus crystals may include about 85 weight percent everolimus and about 15 weight percent ATHC.

In some examples, another excipient, such as, but not limited to, ethyl cellulose, polycaprolactone, and/or poloxamer may be included in the drug coating 34, 134. In some instances, the encapsulated everolimus crystals may include about 80 weight percent everolimus and about 20 weight percent excipient, with the excipient being a combination of ATHC and PCL or a combination of ATHC and poloxamer. As an example, the excipient may be about equal parts of ATHC and PCL or equal parts of ATHC and poloxamer. In other examples, the excipient may have a weight ratio of in the range of about 1:10 ATHC to PCL to about 10:1 ATHC to PCL. In yet other examples, the excipient may have a weight ratio of in the range of about 1:10 ATHC to poloxamer to about 10:1 ATHC to poloxamer. The final coating composition may have a weight ratio of 8:1:1 of therapeutic agent to ATHC to PCL. In another example, the excipient may be more than half ATHC and less than half EC. As an example, the excipient may include two parts ATHC and one part EC.

In some instances, the first pre-mix may include a singular solvent, and preparing the first pre-mix may include placing the solvent in a suitable container and adding the crystalline everolimus and the ATHC. When the solvent includes a mixture of materials, preparing the first pre-mix may include combining or mixing two or more solvents prior to adding the crystalline everolimus and the ATHC. In some examples, the ATHC may be mixed with the solvent prior to adding the ATHC/solvent pre-mix to the therapeutic agent. This may help prevent the therapeutic agent from fully dissolving in the first pre-mix.

In at least some instances, the solvent used for forming the first pre-mix may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic or alicyclic hydrocarbons such as, but not limited to, hexane, heptane, cyclohexane, or the like; aromatic hydrocarbons, such as, but not limited to, toluene or xylenes; and ester solvents such as, but not limited to, ethyl acetate. Mixed solvents such as, but not limited to, ethyl acetate/heptane, acetone/water, IPA/water, IPA/THF, methanol/water, IPA/heptane, or THF/heptane can also be used, for example. Other solvent systems are also contemplated. In some cases, the solvent used for forming the first pre-mix may include cyclohexane.

In some embodiments, the process of forming the first pre-mix may take place at ambient temperature and at atmospheric pressure. In some instances, the first pre-mix may be formed as part of the process of converting the amorphous everolimus into crystalline everolimus. Alternatively, or additionally, ATHC may be added to the suspension that results from the crystallization process, for example. In yet other examples, the first pre-mix may be formed by first mixing the ATHC with the solvent(s) and subsequently adding the crystalline everolimus (or adding the ATHC/solvent pre-mix to the crystalline everolimus).

In some instances, the first pre-mix may include crystalline everolimus suspended in a solvent such as cyclohexane. Alternatively, other solvents such as isopropanol, ethanol or similar alcohols, acetone, tetrahydrofuran, or acetonitrile may be used. In some instances, the first pre-mix may also include other additives such as, but not limited to, BHT, for example. BHT may prevent degradation of the therapeutic agent prior to deploying the coating 34, 134 in the body. It will be appreciated that the crystalline everolimus may minimally dissolve in the solvent, and thus will be a suspension. As such, the crystalline everolimus may maintain its crystal size when suspended in the first solvent. The ATHC should at least partially dissolve in the solvent.

In some instances, the second pre-mix may include a singular solvent without additional excipients and preparing the second pre-mix may include placing the solvent in a suitable container. When the solvent includes a mixture of materials, preparing the second pre-mix may include combining or mixing two or more solvents. It is contemplated that in some embodiments, the second pre-mix may include adding polycaprolactone or poloxamer to the solvent such that the final drug coating 34, 134 includes the therapeutic agent, ATHC, polycaprolactone, and, optionally, BHT or the therapeutic agent, ATHC, poloxamer, and, optionally, BHT.

In at least some instances, the solvent used for forming the second pre-mix may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic hydrocarbons such as, but not limited to, hexane, heptane, or the like; aromatic hydrocarbons, such as, but not limited to, toluene or xylenes; and ester solvents such as, but not limited to, ethyl acetate. Mixed solvents such as ethyl acetate/heptane, acetone/water, IPA/water, methanol/water, IPA/heptane, or THF/heptane can also be used, for example. Other solvent systems are also contemplated. In some cases, the solvent used for forming the second pre-mix may include ethyl acetate.

In some instances, the process of forming the second pre-mix may take place at ambient temperature and at atmospheric pressure. The second pre-mix may include one or more solvents. In some instances, the second pre-mix may include a solvent such as ethyl acetate, although other solvents such as heptane or similar alkanes, or even water, may be used. In some instances, another excipient, such as, but not limited to, ethyl cellulose, polycaprolactone, or poloxamer may added to the second pre-mix.

It is contemplated that at least one of the first or second pre-mixes may include a polar solvent and the other of the first or second pre-mixes may include a non-polar solvent. For example, the first pre-mix may include cyclohexane which is a non-polar solvent, and the second pre-mix may include ethyl acetate which is a polar solvent. The combination of a polar and a non-polar solvent may help to disperse the everolimus crystals. A dispersion of the everolimus crystals may help to dispense controlled, consistent amounts on the medical device to yield a drug coating composition. The organic solvents (e.g., cyclohexane and ethyl acetate in the example given above) are evaporated during the coating manufacturing process. Thus, the drug coating 34, 134 on the medical device may include a drug, one or more excipients, and, optionally, an antioxidant.

In some cases, after the first pre-mix and the second pre-mix have been formed, the second pre-mix may be added to the first pre-mix. In another example, the crystalline everolimus may be added to a container, then the first pre-mix including ATHC, and any other additives, may be added to the container including the everolimus. Next, the second pre-mix (or second solvent) may be added to the everolimus/ATHC mixture. As a result, the ATHC coats the individual crystals of everolimus, resulting in encapsulated everolimus crystals. In some cases, the process may occur quickly. In some cases, the process may occur more slowly. The first pre-mix may include a volume of cyclohexane and the second pre-mix may include a volume of ethyl acetate to provide a ratio of ethyl acetate to cyclohexane in the range of about 1 to 4 to about 1 to 10 when the second pre-mix is added to the first pre-mix. It will be appreciated that having too much everolimus solubility will dissolve the everolimus crystals. It will be appreciated that the stated process of forming the first pre-mix, forming the second pre-mix and then combining the first pre-mix and the second pre-mix is merely illustrative, and other processes are contemplated.

### PROCESS FOR FORMING CRYSTALLINE EVEROLIMUS

Some example processes for converting an amorphous form of a drug into a crystalline form may generally include (a) preparing a suitable solvent, (b) preparing a nucleation initiator with the solvent, (c) combining/mixing the nucleation initiator with an amorphous form of a drug to form a drug precursor dispersion/suspension and (d) incubating the drug precursor dispersion/suspension to allow the amorphous form of the drug to convert to the crystalline form of the drug. The use of a suitable surfactant with the drug precursor dispersion/suspension may allow for the formation of drug crystals having a desirable morphology, allow for the formation of drug crystals having a desirable size and/or shape and/or aspect ratio, allow for the formation of coating suspensions with desirable stability, combinations thereof, and/or the like. Thus, at least some of the processes for converting an amorphous form of a drug into a crystalline form include (a) preparing a suitable solvent, (b) preparing a surfactant solution by combining/mixing the surfactant with the solvent, (c) preparing a nucleation initiator with the solvent, (d) combining/mixing the nucleation initiator with the surfactant solution and with an amorphous form of a drug to form a drug precursor dispersion/suspension and (e) incubating the drug precursor dispersion/suspension to allow the amorphous form of the drug to convert to the crystalline form of the drug. Other processes are also contemplated for converting the amorphous form of the drug into the crystalline form of the drug.

As indicated herein, example processes for converting an amorphous form of a drug into a crystalline form include preparing a suitable solvent. When the solvent is a singular material, preparing a suitable solvent may include placing the solvent in a suitable container. When the solvent is a mixture of materials, preparing a suitable solvent may include combining or mixing the solvents.

In at least some instances, the solvent may include alcohols such as, but not limited to, methanol, ethanol (EtOH), isopropanol (IPA), n-butanol, isobutyl alcohol, or t-butyl alcohol; acetonitrile (ACN); ethers such as, but not limited to, tetrahydrofuran (THF), isopropyl ether (IPE), diethyl ether (DEE); ketone solvents such as, but not limited to, acetone, 2-butanone (MEK), or methyl isobutyl ketone (MIBK); halogenated solvents such as, but not limited to, dichloromethane (DCM), monofluorobenzene (MFB), α,α,α-trifluorotoluene (TFT), nitromethane (NM), ethyl trifluoroacetate (ETFA); aliphatic hydrocarbons such as, but not limited to, hexane, heptane, or the like; aromatic hydrocarbons, such as, but not limited to, toluene or xylenes; and ester solvents such as, but not limited to, ethyl acetate. Mixed solvents such as ethyl acetate/heptane, acetone/water, IPA/water, IPA/THF, methanol/water, IPA/heptane, or THF/heptane can also be used, for example.

When mixtures of solvents are utilized, the mixture may have a suitable ratio of each material. For example, some example solvents may include a mixture of ethyl acetate and heptane. The ratio of ethyl acetate to heptane may be in the range of about 1:4 to 1:30, or about 1:4 to 1:20, or about 1:20. Other mixtures and/or ratios are contemplated. Thus, in some instances, the process for converting an amorphous form of a drug into a crystalline form may include preparing a solvent, for example by mixing ethyl acetate with heptane.

The process for converting an amorphous form of a drug into a crystalline form may include preparing a surfactant solution (e.g., preparing a surfactant solution with the solvent). In at least some instances, the surfactant may include TWEEN 20^{™} (e.g., polysorbate 20, polyoxyethylene (20) sorbitan monooleate, or PEG (20) sorbitan monooleate), TWEEN 80^{™} (e.g., polysorbate 80, polyoxyethylene (80) sorbitan monooleate, or PEG (80) sorbitan monooleate), SPAN^{™} 80, SPAN^{™} 20, TRITON^{™} X-100, TRITON^{™} 400, a non-ionic surfactant, and/or the like. The surfactant solution may have a suitable concentration. For example, the concentration of the surfactant solution may be about 0.01-1% (by weight), about 0.05-0.5% (by weight), or about 0.1% (by weight).

The process for converting an amorphous form of a drug into a crystalline form may include preparing a nucleation initiator (e.g., preparing a nucleation initiator with the solvent). This process or step may also be termed "seeding". In at least some instances, the nucleation initiator may include a crystalline form of the drug suspended in a suitable solvent (e.g., which may or may not be the same solvent that is used to dissolve the amorphous form of the drug). When the drug utilized is everolimus, the nucleation initiator may include a suitable quantity of crystalline everolimus in a suitable solvent. The concentration of the crystalline everolimus in the solvent may be in the ratio of about 0.1-10%, or about 0.1-2%, or about 0.5%. The crystalline everolimus may include everolimus microcrystals formed as described herein. Alternatively, the crystalline everolimus may include everolimus crystals having a different morphology.

The process for converting an amorphous form of a drug into a crystalline form may include mixing and/or combining the nucleation initiator with the surfactant solution and with an amorphous form of a drug to form a drug precursor dispersion/suspension. The amount of the amorphous form of the drug added may vary. For example, about 0.5-100 milligrams of everolimus may be added/dispersed per milliliter (e.g., per milliliter of solvent), or about 1-50 milligrams of everolimus may be added/dispersed per milliliter, or about 10-30 milligrams of everolimus may be added/dispersed per milliliter, or about 20 milligrams of everolimus may be dissolved per milliliter. In at least some instances, the drug dispersion solution may be termed and/or resemble a slurry.

The drug precursor dispersion/suspension may be incubated so that the amorphous form of the drug may convert to the crystalline form. In some instances, incubation may occur over a suitable time period on the order of a number of hours to a number of days. For example, incubation may occur over 24 hours. In some of these and in other instances, incubation may include a high-temperature or warm-temperature incubating step at a suitable temperature (e.g., at about 20°C to 80°C, or at about 40°C to 60°C, or at about 50°C). In some instances, the high-temperature incubating may include agitating the drug precursor dispersion (e.g., using a suitable device such as an orbital shaker). However, in other instances, the high-temperature incubating step is free from agitating/agitation. The high-temperature incubating step may occur over a suitable time period on the order of a number of hours to a number of days. For example, incubation may occur over approximately two days. In some of these and in other instances, incubation may also include a low-temperature or cool-temperature incubating step at a suitable temperature (e.g., at about -10°C to 10°C, or at about 0°C to 5°C, or at about 4°C). The low-temperature incubating step may occur over a suitable time period on the order of a number of hours to a number of days. For example, incubation may occur over several days.

In some instances, the process for converting an amorphous form of a drug into a crystalline form may include one or more of (a) filtering the crystalline form of the drug, (b) washing the crystalline form of the drug, and (c) drying the crystalline form of the drug. For example, some processes are contemplated that include filtering, washing, and drying the crystalline form of the drug. When doing so, the solvent, the surfactant, or both may be essentially completely removed from the drug crystals.

The crystalline form of the drug (e.g., the crystalline form of everolimus) formed by this process may result in crystals with a morphology, size, and shape that allow the formed crystals to be described as being microcrystals. In particular, the use of a surfactant in the crystallization process, along with a suitable solvent mixture, mixed at a suitable ratio, results in the formation of microcrystals. For the purposes of this disclosure, microcrystals may be understood to be crystals that could be described as relatively flat, thin sheets. Some example dimensions may include microcrystals having a width less than about 3 micrometers (e.g., having a non-zero width that is less than about 3 micrometers), a thickness less than about 1 micrometer (e.g., having a non-zero thickness that is less than about 1 micrometer), and a length less than about 10 micrometers (e.g., having a non-zero length that is less than about 10 micrometers). Microcrystals may be desirable for a number of reasons. For example, in some crystallization processes that form "larger", rod-like, or "non-micro" crystals, the resultant crystals can rapidly settle when suspended in a coating material/dispersion. This may make it more challenging to apply the drug crystals to a medical device. The microcrystals formed by the process described herein can be, for example, suspended in a coating material/dispersion and remain in suspension for an extended period of time (e.g., on the order of months or longer).

### EXPERIMENTAL RESULTS

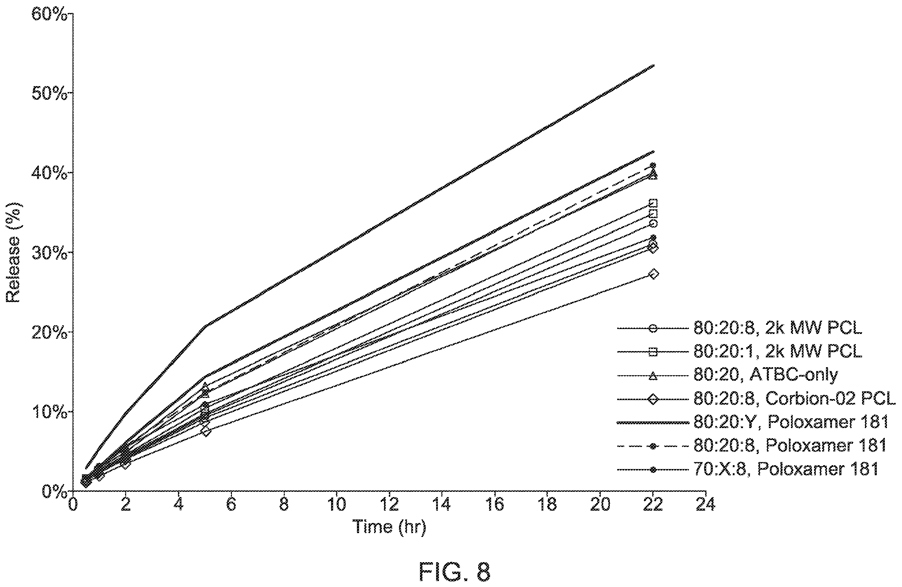

FIG. 8 is a graphical representation of experimental data providing relative drug release rates when using ATBC alone as an excipient, using a combination of ATBC and poloxamer as an excipient, and using a combination of ATBC and PCL as an excipient. The tested compositions are provided in Table 1 below. FIG. 8 shows drug release data for two samples that include crystalline everolimus that is encapsulated with ATBC only, shown graphically as a solid line with triangles. Other samples include crystalline everolimus that is encapsulated with ATBC and PCL, shown graphically as solid lines with a circle, square, or diamond. Additional samples include crystalline everolimus that is encapsulated with a mixture of ATBC and poloxamer, shown graphically as a heavy solid line, a solid line with a solid circle, or a dashed line with a solid circle. It can be seen that the samples with only ATBC as the excipient have eluted about forty percent of the original quantity of everolimus after a period of about twenty hours. In comparison, the samples which include both PCL and ATBC as excipients and some of the samples which include both poloxamer and ATBC have only eluted about twenty five percent to about 35 percent of the original quantity of everolimus after a period of about twenty hours.

Since the combination of both ATBC and PCL and some combinations of ATBC and poloxamer resulted in a loss of less drug over twenty hours, relative to using ATBC alone, and since each composition included the same total amount of excipient, it is appropriate to state that using a combination of ATBC and PCL or ATBC and poloxamer as the excipient may be considered as providing a synergistic improvement in drug release rate.

### TESTED COMPOSITIONS

| **Sample** | **Everolimus (g)** | **ATBC (g)** | **BHT (mg)** | **Poloxamer (g)** | **PCL (g)** | **Cyclohexane (ml)** | **Ethyl acetate (ml)** |
|---|---|---|---|---|---|---|---|
| 1 | 0.311 | 0.078 | 0.62 | 0 | 0.031 | 4 | 1 |
| 2 | 0.311 | 0.078 | 0.62 | 0 | 0.031 | 4 | 1 |
| 3 | 0.309 | 0.077 | 0.62 | 0 | 0.004 | 4 | 1 |
| 4 | 0.309 | 0.077 | 0.62 | 0 | 0.004 | 4 | 1 |
| 5 | 0.309 | 0.077 | 0.62 | 0 | 0 | 4 | 1 |
| 6 | 0.309 | 0.077 | 0.62 | 0 | 0 | 4 | 1 |
| 7 | 0.311 | 0.078 | 0.62 | 0 | 0.031 | 4 | 1 |
| 8 | 0.311 | 0.078 | 0.62 | 0 | 0.031 | 4 | 1 |
| 9 | 0.309 | 0 | 0.62 | 0.077 | 0 | 4 | 1 |
| 10 | 0.309 | 0 | 0.62 | 0.077 | 0 | 4 | 1 |
| 11 | 0.311 | 0.078 | 0.62 | 0.031 | 0 | 4 | 1 |
| 12 | 0.316 | 0.135 | 0.62 | 0.036 | 0 | 4 | 1 |

### TESTING PROCEDURE

A testing solution was made by dissolving Brij^{™} (a nonionic polyoxyethylene surfactant) in a sodium acetate buffer to form a test solution that is a 10mM acetate buffer including 0.2 weight percent Brij^{™}. A coated balloon was placed in a bottle and filled with the test medium at 37°C, and was shaken at 120 rpm. The test solution was sampled at multiple time points (t=0.5 hours, 1 hour, 2 hours, 5 hours, and 24 hours. The collected samples were analyzed on HPLC (high performance liquid chromatography) to measure drug concentration, as the drug present in the test medium represents drug that has eluted from the coating on the balloon. The resulting concentrations were graphed, as seen in FIG. 8.

The materials that can be used for the various components of the medical devices described herein may include those commonly associated with medical devices. The medical devices described herein may include components that may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803, which are incorporated herein by reference. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the medical devices described herein may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the medical devices described herein in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the medical devices described herein to achieve the same result.

In some embodiments, a degree of Magnetic Resonance Imaging (MRI) compatibility is imparted into the medical devices described herein. For example, the medical devices described herein, or portions thereof, may be made of a material that does not substantially distort the image and create substantial artifacts (i.e., gaps in the image). Certain ferromagnetic materials, for example, may not be suitable because they may create artifacts in an MRI image. The medical devices described herein, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

## Claims

1. An elutable coating composition comprising:
a mixture of excipients comprising a bioresorbable polymer or a thermo-gelling polymer and a plasticizing agent; and
a therapeutic agent;
wherein the bioresorbable polymer or the thermo-gelling polymer is selected from a polycaprolactone (PCL) or poloxamer,
wherein the plasticizing agent comprises acetyl tri-butyl citrate (ATBC) or acetyl trihexyl citrate (ATHC), and
wherein the therapeutic agent comprises about 60 to about 90 weight percent of the elutable coating composition, the bioresorbable polymer or the thermo-gelling polymer comprises about 5 to about 20 weight percent of the elutable coating composition, and the plasticizing agent .comprises about 5 to about 20 weight percent of the elutable coating composition.

2. The elutable coating composition of claim 1, wherein the mixture of excipients comprises polycaprolactone.

3. The elutable coating composition of claim 2, wherein the polycaprolactone has a molecular weight of about 10,000 grams/mole (g/mol) or less.

4. The elutable coating composition of any one of claims 1-3, wherein the mixture of excipients comprises approximately equal parts polycaprolactone to acetyl tri-butyl citrate (ATBC).

5. The elutable coating composition of claim 1, wherein the mixture of excipients comprises a poloxamer.

6. The elutable coating composition of claim 5, wherein the poloxamer comprises poloxamer 181 or poloxamer 188.

7. The elutable coating composition of any one of claims 1 or 5-6, wherein the mixture of excipients comprises approximately equal parts poloxamer to acetyl tri-butyl citrate (ATBC).

8. The elutable coating composition of any one of claims 1-7, wherein the therapeutic agent comprises everolimus.

9. The elutable coating composition of any one of claims 1-8, further comprising an antioxidant.

10. A medical device comprising a medical device component that is at least partially covered by a layer of the elutable coating composition in accordance with claim 1.

11. The medical device of claim 10, wherein the medical device component is an expandable balloon or an expandable stent.

## Patentansprüche

1. Eine eluierbare Beschichtungszusammensetzung, umfassend:
ein Gemisch aus Exzipienten, umfassend ein bioresorbierbares Polymer oder ein thermogelierendes Polymer und einen Weichmacher; und
einen Wirkstoff;
wobei das bioresorbierbare Polymer oder das thermogelierende Polymer ausgewählt ist aus Polycaprolacton (PCL) oder Poloxamer,
wobei der Weichmacher Acetyl-tributylcitrat (ATBC) oder Acetyl-trihexylcitrat (ATHC) umfasst, und
wobei der Wirkstoff etwa 60 bis etwa 90 Gewichtsprozent der eluierbaren Beschichtungszusammensetzung umfasst, das bioresorbierbare Polymer oder das thermogelierende Polymer etwa 5 bis etwa 20 Gewichtsprozent der eluierbaren Beschichtungszusammensetzung umfasst und der Weichmacher etwa 5 bis etwa 20 Gewichtsprozent der eluierbaren Beschichtungszusammensetzung umfasst.

2. Die eluierbare Beschichtungszusammensetzung gemäß Anspruch 1, wobei das Gemisch aus Exzipienten Polycaprolacton umfasst.

3. Die eluierbare Beschichtungszusammensetzung gemäß Anspruch 2, wobei das Polycaprolacton ein Molekulargewicht von etwa 10.000 Gramm/Mol (g/mol) oder weniger aufweist.

4. Die eluierbare Beschichtungszusammensetzung nach einem der Ansprüche 1 bis 3, wobei das Gemisch aus Exzipienten etwa gleiche Anteile an Polycaprolacton und Acetyl-tributylcitrat (ATBC) umfasst.

5. Die eluierbare Beschichtungszusammensetzung gemäß Anspruch 1, wobei das Gemisch aus Exzipienten ein Poloxamer umfasst.

6. Die eluierbare Beschichtungszusammensetzung gemäß Anspruch 5, wobei das Poloxamer Poloxamer 181 oder Poloxamer 188 umfasst.

7. Die eluierbare Beschichtungszusammensetzung nach einem der Ansprüche 1 oder 5-6, wobei das Gemisch aus Exzipienten etwa gleiche Anteile an Poloxamer und Acetyl-tributylcitrat (ATBC) umfasst.

8. Die eluierbare Beschichtungszusammensetzung nach einem der Ansprüche 1-7, wobei der Wirkstoff Everolimus enthält.

9. Die eluierbare Beschichtungszusammensetzung gemäß einem der Ansprüche 1-8, umfassend ferner ein Antioxidans.

10. Eine medizinische Vorrichtung, umfassend eine medizinische Vorrichtungskomponente, die zumindest teilweise mit einer Schicht der eluierbaren Beschichtungszusammensetzung gemäß Anspruch 1 beschichtet ist.

11. Die medizinische Vorrichtung gemäß Anspruch 10, wobei die medizinische Vorrichtungskomponente ein expandierbarer Ballon oder ein expandierbarer Stent ist.

## Revendications

1. Composition de revêtement éluable comprenant :
un mélange d'excipients comprenant un polymère biorésorbable ou un polymère thermogélifiant et un agent plastifiant ; et
un agent thérapeutique ;
dans laquelle le polymère biorésorbable ou le polymère thermogélifiant est choisi parmi une polycaprolactone (PCL) et un poloxamère,
dans laquelle l'agent plastifiant comprend du citrate d'acétyltributyle (ATBC) ou du citrate d'acétyltrihexyle (ATHC), et
dans laquelle l'agent thérapeutique représente environ 60 à environ 90 % en poids de la composition de revêtement éluable, le polymère biorésorbable ou le polymère thermogélifiant représente environ 5 à environ 20 % en poids de la composition de revêtement éluable, et l'agent plastifiant représente environ 5 à environ 20 % en poids de la composition de revêtement éluable.

2. Composition de revêtement éluable selon la revendication 1, dans laquelle le mélange d'excipients comprend de la polycaprolactone.

3. Composition de revêtement éluable selon la revendication 2, dans laquelle la polycaprolactone a une masse moléculaire d'environ 10 000 grammes/mole (g/mol) ou moins.

4. Composition de revêtement éluable selon l'une quelconque des revendications 1 à 3, dans laquelle le mélange d'excipients comprend des parties approximativement égales de polycaprolactone et de citrate d'acétyltributyle (ATBC).

5. Composition de revêtement éluable selon la revendication 1, dans laquelle le mélange d'excipients comprend un poloxamère.

6. Composition de revêtement éluable selon la revendication 5, dans laquelle le poloxamère comprend du poloxamère 181 ou du poloxamère 188.

7. Composition de revêtement éluable selon l'une quelconque des revendications 1, 5 et 6, dans laquelle le mélange d'excipients comprend des parties approximativement égales de poloxamère et de citrate d'acétyltributyle (ATBC).

8. Composition de revêtement éluable selon l'une quelconque des revendications 1 à 7, dans laquelle l'agent thérapeutique comprend de l'évérolimus.

9. Composition de revêtement éluable selon l'une quelconque des revendications 1 à 8, comprenant en outre un antioxydant.

10. Dispositif médical comprenant un composant de dispositif médical qui est au moins partiellement recouvert d'une couche de la composition de revêtement éluable selon la revendication 1.

11. Dispositif médical selon la revendication 10, dans lequel le composant de dispositif médical est un ballonnet expansible ou une endoprothèse expansible.
